# EUROPEAN PATENT APPLICATION

(11) **EP 1 764 100 A2**
(43) Date of publication of application: **21.03.2007**
(21) Application number: 06254711.2
(22) Date of filing: 11.09.2006
(51) Int. Cl.: A61K 31/4174, A61K 9/00, A61K 9/02, A61K 47/12, A61K 47/14, A61K 47/32, A61K 47/38, A61P 15/02

(54) **Compositions and methods for reducing vaginal pH**

(30) Priority: 12.09.2005 US 224189; 13.09.2005 US 224870
(71) Applicant: JOHNSON & JOHNSON CONSUMER COMPANIES, INC., Skillman, New Jersey 08558 (US)
(72) Inventor: Ahmad, Nawaz, Monmouth Junction, NJ 08852 (US); Cui, Cheng-Ji, Penington, NJ 08534 (US); Fu, Ann, Bridgewater, NJ 08807 (US); Lin, Shun Y., Plainsboro, NJ 08536 (US)
(74) Representative: Fisher, Adrian John

(57) **Abstract**

This invention relates to aqueous buffered compositions and methods for lowering vaginal pH and reducing the level of self-perceived vaginal odor. Such compositions may be applied to the vaginal area to lower vaginal pH and assist in maintaining such pH over a period of time.

## Description

This application is a continuation-in-part application of U.S. Patent Applications Serial Numbers 10/ 109,097 and 10/ 128,611 filed respectively on March 28, 2002 and April 23, 2002, as well as Serial Number (Attorney Docket File No. PPC833CIP2) filed September 12, 2005 and hereby incorporates by reference all subject matter set forth therein.

### Field of the Invention

This invention relates to compositions and methods for maintaining healthy vaginal pH in order to control vaginal odor.

### Background of the Invention

Bacterial vaginosis is a change in flora, the cause of which is still unknown in the vast majority of instances. Bacterial vaginosis has generally been used to represent any change in vaginal flora resulting in an assumed loss of lactobacilli. However, whether such flora represent the genetically normal state of the vagina in all women is poorly defined. Most therapies recommended for bacterial vaginosis in non-pregnant women are often successful in the short term, but usually unsuccessful if long-term follow-up is conducted. Although bacterial vaginosis is generally believed to be an endogenous condition, a number of behavioral factors are possibly involved, such as the use of contraceptive and intimate hygiene products and lifestyle habits. Although bacterial vaginosis is not considered a true sexually transmitted infection, it may be correlated to multiple sexual partners. Therefore, there is an increasing need to develop a product that is effective against bacterial vaginosis and other vaginitis.

Normal vaginal pH ranges from 3.8 to 4.5. At this pH, the microbial flora which inhabit the healthy vagina, including lactobacilli, thrive. Furthermore, at this pH, opportunistic bacterial growth and attachment to the vaginal walls is inhibited. One important function that lactobacilli perform is modulating normal vaginal flora. They accomplish this both by producing lactic acid, which maintains an acidic pH and by producing hydrogen peroxide, which inhibits catalase-negative bacteria.

Certain activities and conditions can raise vaginal pH, including menstruation, intercourse and lack of estrogen, such as occurs in menopause. An elevated pH is also part of the differential diagnosis of bacterial vaginosis.

In women with bacterial vaginosis, there is a seven-fold decrease in the prevalence of facultative *Lactobacilli* and a corresponding increase in the population of other bacteria, some of which may be pathogenic. Symptomatic women with bacterial vaginosis have a 100-to 1000-fold increase in *Gardenerella vaginalis, Mobiluncus, Peptostreptococcus and Bacteroides.* Besides pruritus, yellow discharge and irritation, the most common symptom of vaginosis is odor, specifically an amine odor. The characteristic amine odor in bacterial vaginosis is created by products from anaerobic bacterial metabolism in the vaginal fluid of women with bacterial vaginosis. The elevation of pH volatizes amines from protein attachment and produces the odor.

It is therefore may be desirable for individuals to restore "healthy" vaginal pH whenever an increase in the vaginal pH is observed. Not only would this restoration appear to support or promote the maintenance of a healthy selection of vaginal flora, it may assist in controlling odor that may be caused by the growth of pathogenic bacteria.

U.S. Patent Number 6,017,521 (Robinson et al.) describes the use of water-swellable, water-insoluble carboxylic acid polymers in aqueous compositions that may be administered to the vagina to lower pH of the vagina. Carboxylic acid polymers such as carbopols and carbomers, are high molecular weight, cross-linked, acrylic acid-based polymers. Carbopols have been extensively used as gelling and viscosity building agents in pharmaceutical and cosmetic applications. The pH of a 1% water dispersion of these Carbopol polymers is generally between about 2.5 and about 3. The molecules of these acrylic acid polymers are highly coiled in conformation, thus limiting their solubility and thickening capability. Carbopols tend to be water insoluble and water swellable. When dispersed in water, the molecule begins to hydrate and uncoil slightly, generating an increase in viscosity. In order to achieve the highest possible performance with the polymer, the molecule should be completely uncoiled. As the molecule uncoils, its solubility increases. Generally, neutralizing the polymer with a suitable base like sodium or potassium hydroxide is a method used to uncoil these molecules, a rapid reaction that instantaneously thickens the composition in which the polymer resides. Although Carbopol polymers can be used without neutralization, neutralization aids in solubility.

However, Robinson does not provide an anhydrous composition that allows the polymer to become neutralized when exposed to a relatively basic environment.

Organic acids such as acetic, sorbic, lactic, citric, tartaric acid and the like used alone and applied to the vagina will lower the but will not maintain a low pH because of they have a low buffering capacity.

### Brief Description of the Drawings

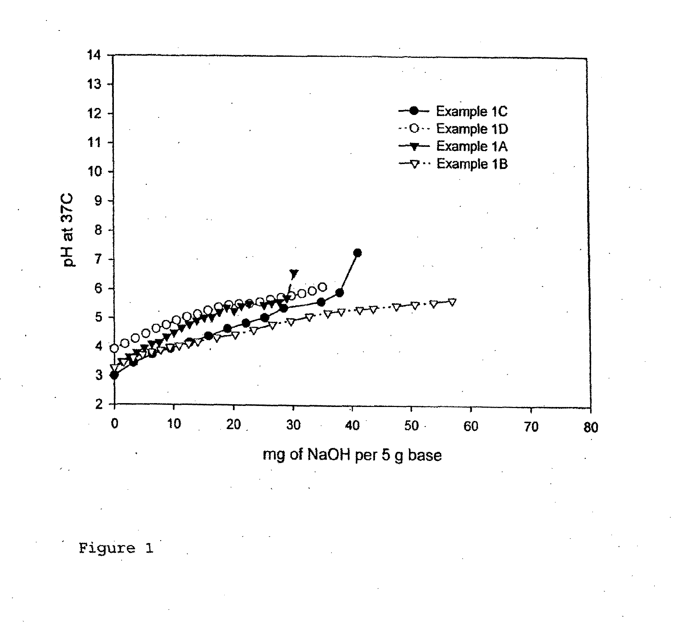

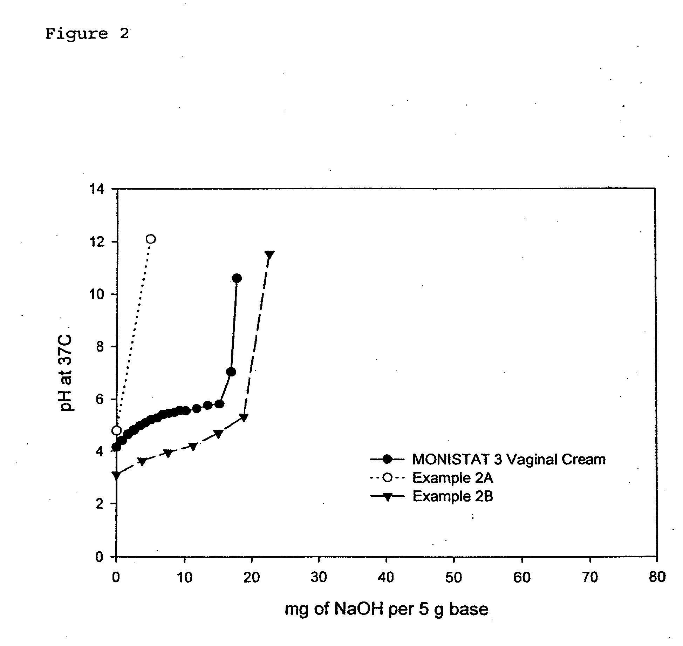

Figures 1 and 2 demonstrate the buffer capacity for the aforementioned cream formulations of Examples 1 and 2. Monistat 3® vaginal cream is used as a control. As shown, Examples 1A, 1B, 1C, 1D and 2B have relatively good buffering capacity while comparative Example 2A and Monistat 3® vaginal cream do not. Example 2A does not contain either buffer or carbomer. The buffer capacity of cream base is improved significantly after addition of 1.8% or more of glucono delta lactone or a combination of gluconodelta lactone and carbomer. A better buffer capacity is also observed for formulations containing miconazole nitrate as compared with placebo (Example 1C as compared with Example 2B). This is surprising indicating that the miconazole nitrate could enhance the buffer capacity in the described cream formulations.

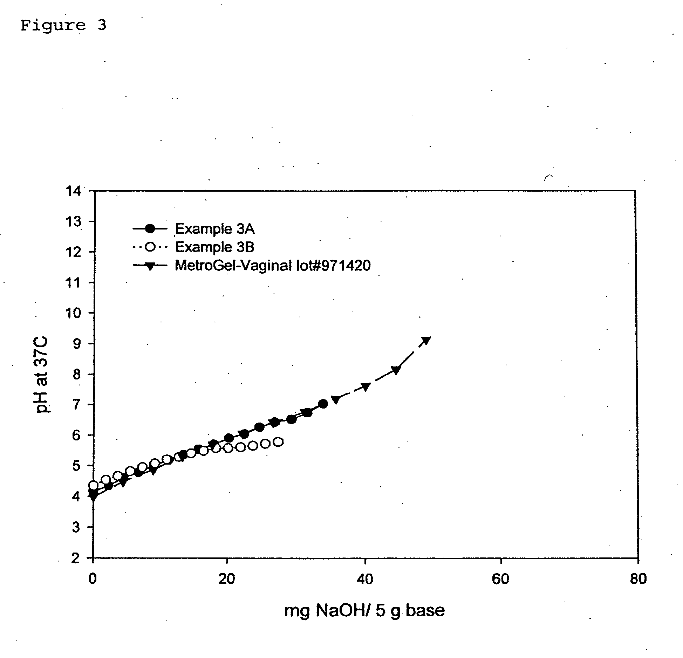

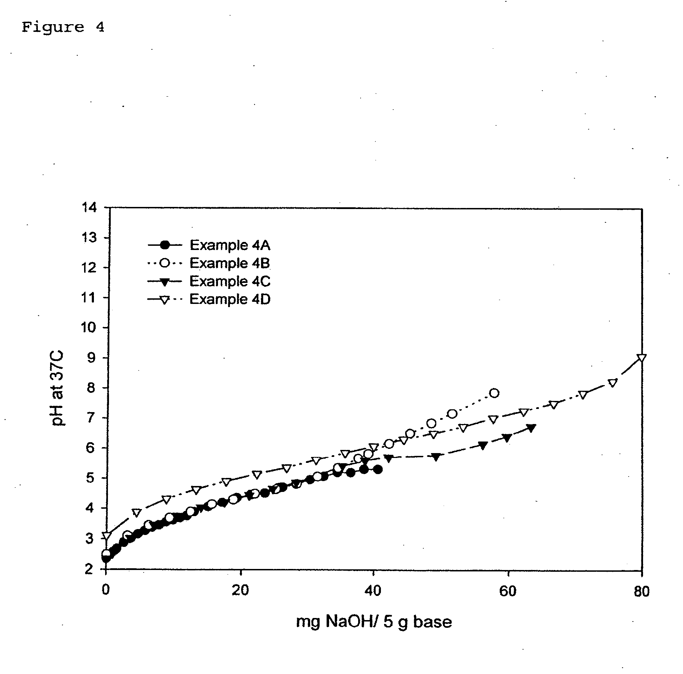

Figures 3 and 4 demonstrate buffered gel formulations of Examples 3 and 4, compared to MetroGel-Vaginal®, a commercial formulation for treating bacterial vaginosis. Formulations 3A, 4B and 4D do not contain miconazole nitrate and have relatively less buffering capacity than the other formulations containing miconazole nitrate (3B, 4A, and 4C).

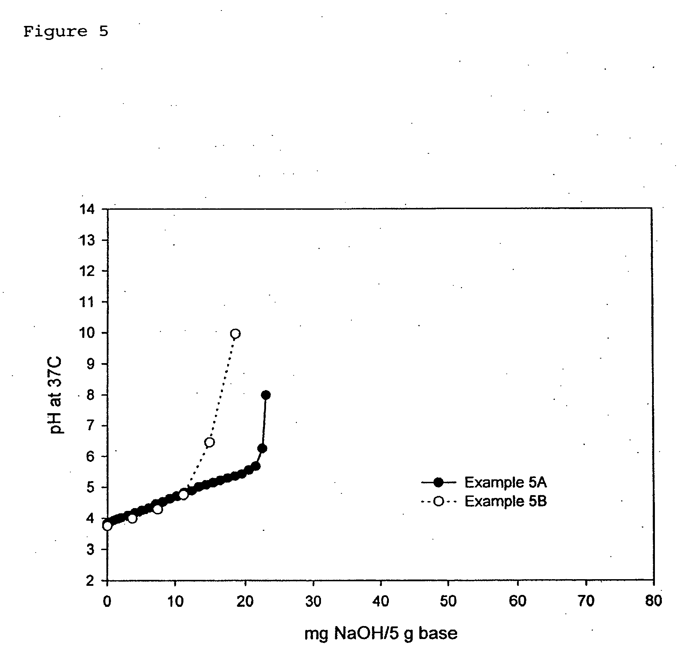

Figure 5 demonstrates buffered gel formulations of Example 5. Formulation 5B does not contain miconazole nitrate and have relatively less buffering capacity than the other examples.

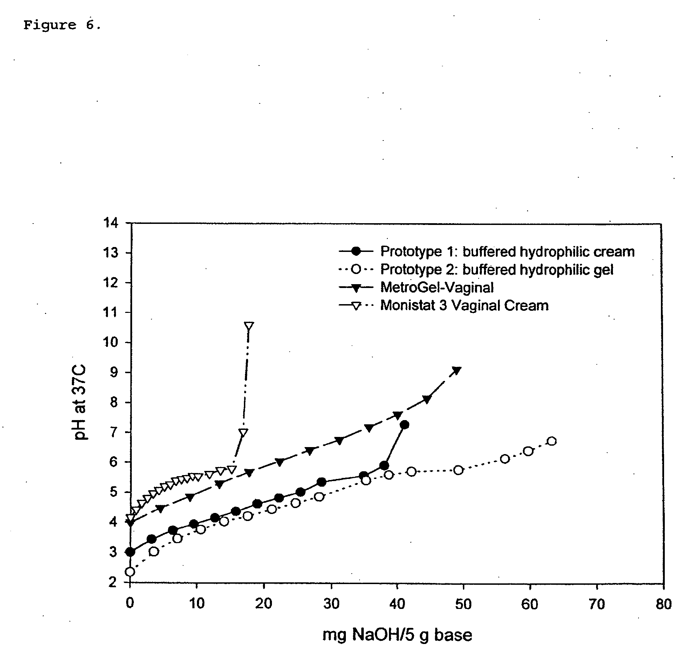

Figure 6 demonstrates a comparison between preferred buffered formulations of this invention, formulations 1C and 4C, compared to MetroGel-Vaginal®, a commercial formulation containing metronidazole for treating bacterial vaginosis locally and Monistat 3® Vaginal Cream, a commercial formulation containing miconazole nitrate for treating vulvovaginal candidiasis locally. As demonstrated, the compositions of this invention are more capable of maintaining a healthy pH by buffering capacity than the commercial products.
FIG. 7 is a graph comparing the buffering capacity of 4% Carbopol 974P in anhydrous base (Composition A), 3% lactic acid solution in the anhydrous base (Composition B) and 4% Carbopol and 3% lactic acid in the anhydrous base with RepHresh® Vaginal Gel.
FIG. 8 is a graph comparing the buffering capacity of composition Example 10A and Example 10B of the compositions of this invention with the RepHresh® Vaginal Gel.
FIG. 9 is a graph comparing the *in vivo* pH effects of applying compositions of this invention, a composition as set forth in co-pending patent application Serial No. (Attorney Docket No. PPC5209 filed concurrently herewith) and RepHresh® Vaginal Gel.

### Summary of the Invention

The compositions and methods of this invention relate to products containing an antifungal compound and an active buffering compound as well as a pharmaceutically acceptable carrier. The buffered compositions of this invention are expected to have surprising effectiveness in treating both mycotic infections and bacterial vaginosis. The pH of the compositions of this invention are preferably maintained between about 2.5 and about 5.5. More preferably, the pH should be maintained between about 3 and about 5, most preferably between about 3 and about 4.5. At this pH range, both the antifungal compounds and the vaginal environment are conducive to treatment and prophylaxis of mycotic and bacterial vaginosis infections.

Buffering agents according to this invention may be applied into the vagina prior to, during, or after an intravaginal antifungal drug treatment. The buffering agents may be co-administered with the antifungal azole in the same composition. They may also be administered as two different or separate compositions, but substantially simultaneously. Alternatively, the respective antifungal azole composition and buffering composition may be administered sequentially and separated by a certain time period.

Thus, the compositions and methods of this invention relate to:
A composition for treating vulvovaginitis and vaginosis comprising:
   a) an antifungal agent; and
   b) a buffering system. More preferably, this invention relates to a composition for treating vulvovaginitis and vaginosis comprising:

   a) an azole antifungal agent; and
   b) a buffering system comprising gluconodeltalactone. This invention also relates to a composition for treating vulvovaginitis and vaginosis comprising:

   a) an azole antifungal agent;
   b) a buffering system;
   c) a pharmaceutically acceptable carrier. The compositions of this invention relate to an emulsion composition for treating vulvovaginitis and vaginosis comprising:

   a) an azole antifungal agent;
   b) a buffering system comprising gluconodeltalactone;
   c) a carbomer; and
   d) a pharmaceutically acceptable carrier; as well as a gel composition for treating vulvovaginitis and vaginosis comprising:

   a) an azole antifungal agent;
   b) a buffering system;
   c) polyethylene glycol; and
   d) a pharmaceutically acceptable carrier. The compositions and methods of this invention also relate to a dual-phase composition for treating vulvovaginitis and vaginosis comprising:

   a) a lipophilic, or oil phase comprising an azole antifungal agent and the lipophilic pharmaceutically acceptable carrier; and
   b) a hydrophilic, or water phase comprising a buffering system and the hydrophilic pharmaceutically acceptable carrier. The methods of this invention relate to a method for treating vulvovaginitis and vaginosis comprising administering to a vaginal mucous membrane a composition comprising an azole antifungal agent and a buffering system. The compositions and methods of this invention may also be useful in preventing, i.e., in the prophylaxis, of vaginal infections in accordance with the compositions and methods set forth above. The compositions of this invention may also be packaged in a kit containing the compositions according to this invention as well as a soothing composition containing anti-irritant, anti-inflammatory, emollients, antifungal, antiseptic and like ingredients which can be applied to the vulvar skin in order to soothe and protect the skin and help it to heal.

The compositions and methods of this invention also relate to compositions containing:
a) a buffering system; and
b) a pharmaceutically acceptable carrier
for use in adjusting and maintaining vaginal pH and reducing perceived vaginal odor.

Surprisingly, although miconazole nitrate is not generally effective against bacterial infections, we have found that its antibacterial activity is significantly enhanced by buffering.

### Detailed Description of the Preferred Embodiments

Vaginal infections such as candidiasis-related infection require an active antifungal compound in the dosage form to treat the infection. Azole-type antifungals are known for effectiveness in treating vaginal mycotic infections without disrupting the vaginal flora. Several azole compounds with proven efficacy against fungal infection have been approved for OTC use, including vaginal products containing miconazole nitrate, tioconazole, or clotrimazole. Therefore, the safety of these azole products has been established.

Although the effectiveness of these VVC effective azole products for treating bacterial vaginosis related infections has not been proven, using the compositions of this invention, there exists an opportunity to develop an effective dosage from these safe antimycotic-effective compounds for vaginal infections such as candidiasis, bacterial vaginosis, and mixed infections.

The novel compositions of this invention, which combine the antimycotic effectiveness of antifungal ingredients with a buffered carrier composition, maintain or adjust the vaginal pH to healthy levels and permit treatment and, potentially, prophylaxis, of both vulvovaginitis and bacterial vaginosis.

The dose of antifungal agent for treating vulvovaginitis and bacterial vaginosis varies depending upon the antifungal active ingredient used and its potency. The amount of the antifungal ingredient effective to treat an infection is referred to as the "therapeutically effective amount". The antifungal agent in the compositions of this invention should preferably be present in a therapeutically effective amount. Preferably, they should be present in an amount from about 0.01 % to about 90% weight by weight of the composition. More preferably, they should be present in an amount from about 0.1% to about 50% weight by weight, more preferably in an amount from about 0.4% to about 10% weight by weight. The buffering agent in the composition should be present in an amount of from about 0.01% to about 50% w/w. More preferably, it should be present in an amount of from about 0.1% to about 20% w/w and most preferably, from about 1 % to about 5% weight by weight.

Other components may be present in the compositions of this invention such as water, anti-oxidants, chelating agents, preservatives, oils, waxes, surfactants, emulsifiers, viscosity building agents, solvents, moisturizing agents, solubilizers and bioadhesives/muco-adhesives and the like. The relative quantities of such components may vary according to the desired nature and consistency of the composition, including creams, ointments, waxy suppositories, gelatin capsules, anhydrous polymeric suppositories and the like.

The preferred buffered forms of the compositions of this invention may be made as emulsions, gels or as two-phase, or dual, dosage forms. Preferably, one hydrophilic phase is present in the compositions of the invention in order to provide a sector of the composition, which can be buffered. Three preferred buffered dosage form designs containing an active antifungal compound against are as follows: i) A hydrophilic cream, ii) hydrophilic gel, iii) and a two-phase dosage form design for treating vaginal infections described above. These would ease consumers' desire for immediate and effective treatment of vaginal infection. The buffer capacity of each formulation is formulated to be able to maintain the pH at a level of from about 3 to about 5.5, more preferably from about 3 to about 4.5.

The buffering agents according to this invention may be applied into the vagina prior to, during, or after an intravaginal antifungal drug treatment. The buffering agents may be co-administered with the antifungal azole in the same composition, or as two different or separate compositions, but administered together or substantially simultaneously. For example, the buffering agents may be incorporated directly into a composition containing an antifungal azole compound. In this case, the buffering agent and the azole compound are preferably administered to patients simultaneously during application. The buffering agents may be coated on the outer surface of an vaginal suppository (e.g., a wax- or fatty acid based antifungal vaginal suppository), or a gelatin capsule suppository containing an antifungal drug. The buffering agents can also be incorporated into the gelatin-wall of the antifungal gelatin capsule.

Surprisingly, we have found that the use of the compositions of this invention that do not contain an antifungal azole have very high buffer capacities and are expected to be able to lower and maintain such lower pH in the vaginal environment. Preferably, compositions containing such buffering agents (hereinafter, "buffering compositions") contain at least one organic acid and one water-soluble polyacrylic acid polymer, although such compositions may contain at least one organic acid or one water-soluble polyacrylic acid polymer. Preferably, the water-soluble polyacrylic acid polymer is in a non-neutralized state.

Alternatively, the respective antifungal azole composition and buffering composition may be administered sequentially and separated by a certain time period. For example, a composition for intravaginal application may contain only the buffering agents without any antifungal azole. Such buffering composition may be administered into the vagina when an antifungal azole is present in the vagina. The azole already in the vagina resulted from an earlier intravaginal or oral antifungal treatment, and the buffering agent(s) work to extend the intended therapeutic efficacy to treat or prevent the occurrence of bacterial vaginosis. Since the antifungal azole in vaginal tissue and fluid usually lasts for several days following an intravaginal or an oral administration, the buffer composition may be administered preferably from less than one hour to about 10 days, more preferably, from about 8 hours to about 7 days, and most preferably, from about 12 hours to about 5 days, after anti-fungal administration.

The dosing regimen will vary depending upon the particular antifungal agent that is being employed in the products of the invention. A therapeutically effective or prophylactically effective dose should be employed.

Alternatively, the buffering agent may be administered before and/or after the intravaginal antifungal treatment. Preferably, buffering agents are incorporated into certain polymeric or biopolymer muco-adhesive materials, such as gelatin, chitosan and its derivatives, hydrophilic cellulose (preferably a hydroxyalkylcellulose and more preferably, hydroxymethylcellulose, hydroxyethylcellulose, or the like or a mixture thereof), and polyacrylate-polyacrylic acid polymers (e.g., Carbomers and the like). The hydrophilic polymer containing buffering agents may serve as gelling agent in a gel-type composition, or viscosity-building agent in an emulsion-type composition as in, for example, an oil-in-water cream.

Alternatively, the buffering agent-embedded hydrophilic polymer may be suspended in a lipophilic composition containing an antifungal drug (for example, an ointment, a wax-/fatty acid-suppository, or a water-in-oil emulsion). Upon application into the vagina, the hydrophilic polymer will adhere to the vaginal mucosal membrane, thus maintaining the vaginal pH at the preferable pH range for a prolonged period of time, even long after the antifungal drug has been eliminated or excreted from the vagina. Such prolonged maintenance of vaginal acidity assures re-establishment of healthy microbial flora (e.g., *Lactobacillus* species), and inhibits opportunistic pathogenic yeast (e.g., *Candida albicans*) in the vagina.

The antifungal compositions of this invention should contain at least one active antifungal ingredient, preferably an azole antifungal ingredient. More preferably, such compounds are miconazole nitrate, terconazole, butaconazole, itraconazole, voriconazole, ketoconazole, econazole, tioconazole, fluconazole, posconazole, ravuconazole, clotrimazole and the like.

The compositions of this invention should also contain at least one buffering system or agent. Preferably, such buffering agent is gluconodeltalactone ("GDL"). GDL is a neutral cyclic ester of gluconic acid. When added into an aqueous solution, GDL rapidly dissolves, and subsequently slowly hydrolyzes to gluconic acid. Other buffering systems or agents may be used as well in the compositions and methods of this invention.

The term "buffer system" or "buffer" as used herein refers to a solute agent or agents which, when in aqueous solution, stabilize such solution against a major change in pH (or hydrogen ion concentration) when acids or bases are added thereto. Solute agent or agents which are used for a resistance to change in pH from a starting buffered pH value around pH 4 as preferably utilized in the compositions and methods of this invention. In general, buffers for the compositions of this invention include any physiologically acceptable organic acid and its corresponding salt, either liquid or solid (depending upon the desired form of application. Preferably, such buffers have a pKa from about pH 3 to about pH 5. Buffers preferably useful in the compositions and methods of this invention include, but are not limited to, acetic, fumaric, lactic, citric, propionic, lactic, malic, succinic, gluconic, ascorbic, tartaric acids and the like.
Polymers with ionizable functional groups, including, for example, a carboxylic acid or an amine group, and a buffering capacity may also be used as polymeric buffers according to this invention. Examples of polymeric buffers preferably used in the compositions and methods of this invention include Carbomer® or Carbopol®, available commercially from B.F. Goodrich Co., Akron, Ohio, and carboxymethyl celluloses. More preferably, in the buffering compositions of this invention, water-soluble polyacrylic polymers should be utilized in a non-neturalized state. Most preferably, water-soluble polyacrylic acid polymers sold under the trade name CARBOPOL® including CARBOPOL® 974, CARBOPOL® 934 and CARBOPOL® 980, commercially available from NOVEON, B.F. Goodrich Chemical Corporation of Cleveland, Ohio. Virtually any pharmaceutically acceptable buffer system that achieve a pH in the preferred range for topical applications may be used in the compositions and methods of this invention.

Buffered formulations of an azole suitable for vaginal application according to the present invention and suitable for achieving the desired therapeutic action and physiological pH of the vagina of about 4 may be formulated in any convenient non-flowing form, including, but not limited to, suspensions, emulsions, clear and opaque gels, semisolid systems, including ointments, pastes, oil-in-water (o/w) creams, semisolid emulsions with solid internal phases, semisolid emulsions with fluid internal phases, vaginal suppositories, insertable tablets, soft or hard gelatin capsules and the like.

The buffering compositions of this invention may also contain polyols in accordance with relative quantities that vary according to the desired nature and consistency of the intended dosage form or application. Preferably, the compositions of this invention contain at least one polyol. Preferably, the polyol is a polyhydric alcohol, and more preferably, at least two polyhydric alcohols. Polyethylene glycol (hereinafter, "PEG") ethers may also be used, including PEG ethers of propylene glycol, propylene glycol stearate, propylene glycol oleate and propylene glycol cocoate and the like. Specific examples of such PEG ethers include PEG-25 propylene glycol stearate, PEG-55 propylene glycol oleate and the like. Preferably, at least one of the polyhydric alcohols of the compositions of this invention is a polyalkylene glycols or others selected from the following group: glycerine, propylene glycol, butylene glycol, hexalene glycol or polyethylene glycol of various molecular weight and the like and/or combination thereof. More preferably, the compositions of this invention contain a polyethylene glycol; most preferably, the polyethylene glycol may be selected from the following group: polyethylene glycol 400 or polyethylene glycol 300. Polypropylene glycol of various molecular weights may also be used. PEGylated compounds such as peptide or protein derivatives obtained through PEGylation reactions may also be used. In addition, block copolymers of PEG's may be used, such as (ethylene glycol)-block-poly(propylene glycol)-block-(polyethylene glycol), poly(ethylene glycol-ran-propylene glycol) and the like. These polyols may be useful in the compositions of this invention as solvents, humectants or carriers.

The buffering compositions of this invention may also include hydrogenated vegetable oils such as hydrogenated palm glyceride (known in the art under the trade name Myverol 18-04), mineral oil and the like. The buffering compositions of this invention may also include cellulosic polymers as viscosity building and enhancing agents and may include but are not limited to alkylcellulose polymers, including, but not limited to, hydroxymethylcellulose, hydroxyethycellulose, hydroxypropycellulose and hydropropylmethylcellulose. Compositions of this invention may preferably include bioadhesive agents such as MVE/MA copolymer such as that sold under the trade name Stabileze 06 and Calcium Sodium PVM/MA copolymer sold under the trade name Gantrez MS-955. The compositions of this invention may also include dl-alpha tocopherol acetate or Vitamin C.

The compositions of this invention may also contain fatty alcohols, more preferably, stearyl alcohol or cetyl alcohol or the like. These fatty alcohols may be useful as emollients or enhancing agents.

Surprisingly, it was found that a buffered gel containing an azole antifungal agent, miconazole nitrate, had a better buffer capacity with a pH of between about 3 and about 5.5 than buffered gels that did not contain miconazole nitrate.

Standard classical buffers known to those of ordinary skill in the art generally contain an acid in combination with a base to maintain a certain pH within a composition. Buffers developed in this manner generally resist changes in the pH and maintain the original pH of the buffer. The extent to which these buffers can resist change in pH is designated as their "buffering capacity". The anhydrous acid-acid buffers of this invention are surprisingly different from the standard buffers known to those of ordinary skill in the art in that they have a substantially high buffering capacity than known classical buffer systems.

The classical method of determination of buffering capacity of a buffer is to titrate the buffer using 1 Normal Sodium Hydroxide Solution and mg of NaOH is calculated per 5g of the test sample. Because the acidic compositions of this invention will not be subjected to an interaction with sodium hydroxide normal human use, we have modified the method for determining buffering capacity in accordance with simulated human conditions. Instead of 1 N sodium hydroxide solution, a standard pH 7 buffer was used for titration. The pH of a 10 g sample of the test article was measured and, to this sample, pH 7 buffer was added in 5 ml increments with continuous mixing while monitoring the resulting pH change. The titration was discontinued when pH reached around 7.

Typical buffer systems of this invention are summarized in Table 1 as examples. The concentration of polyacrylic polymer and organic acid in these buffer systems can be customized in order to deliver a desired pH and buffering capacity, depending on other ingredients and combination of ingredients in the compositions set forth herein.

| **INGREDIENT** | **COMPOSITION A Carbopol Alone** | **COMPOSITION B Lactic Acid Alone** | **COMPOSITION C Carbopol-Lactic Acid Combination** |
|---|---|---|---|
| INGREDIENT | % w/w | % w/w | % w/w |
| Carbopol 974P | 4.00 | | 4.00 |
| Water | 96.00 | 97.00 | 97.00 |
| Lactic Acid | | 3.00 | 3.00 |
| Total | 100.00 | 100.00 | 100.00 |

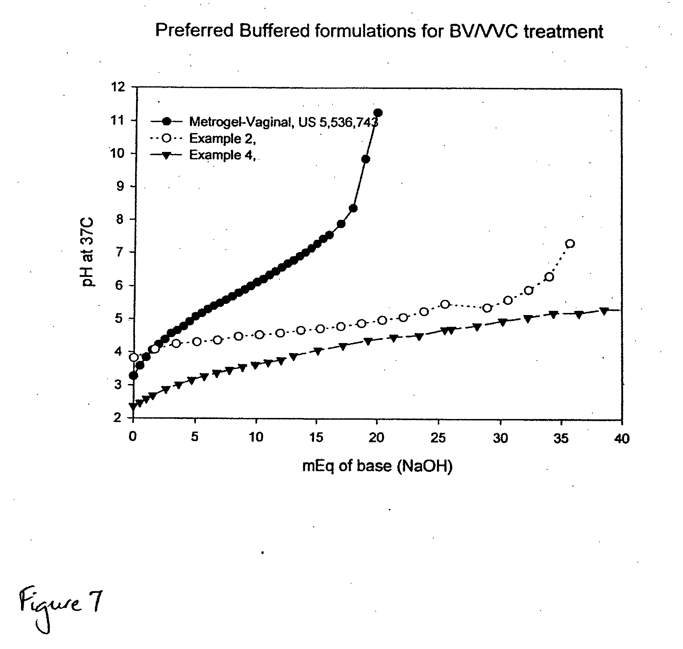

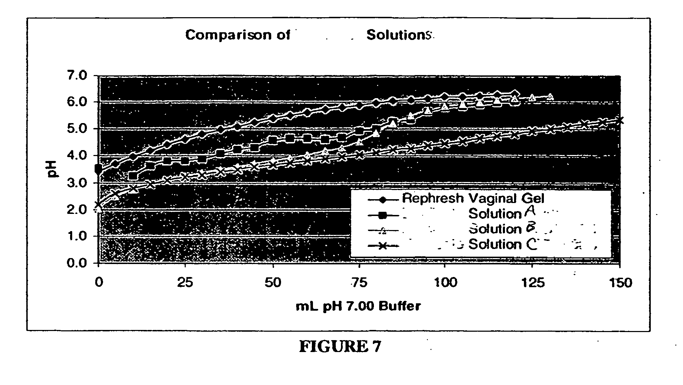

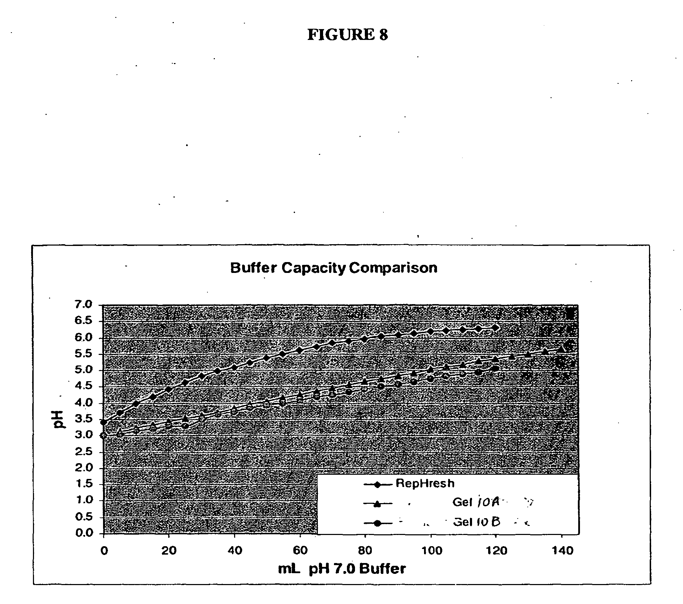

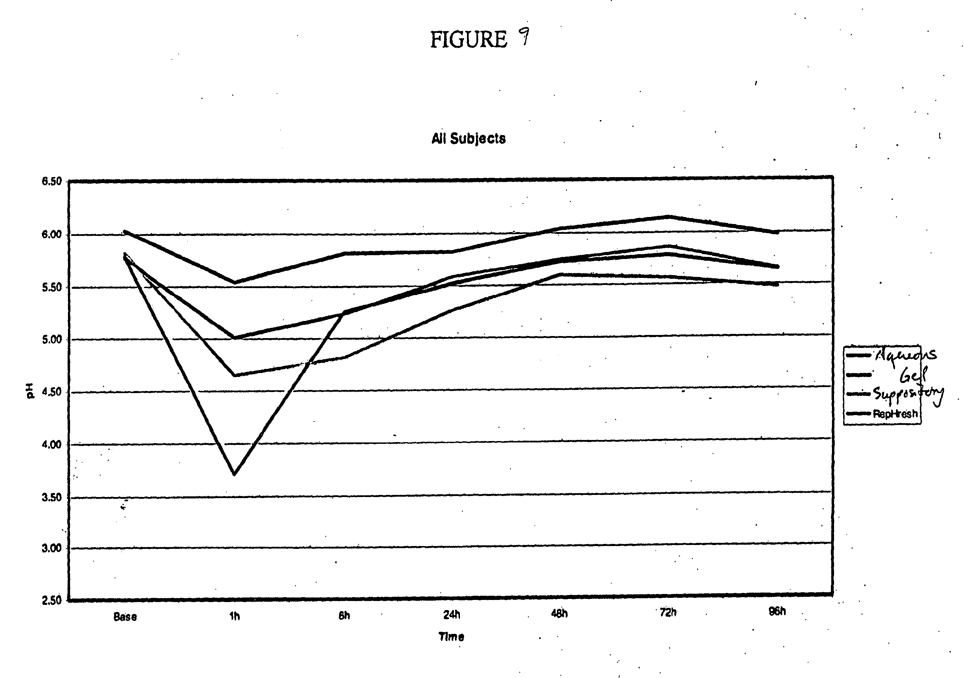

The buffer systems of this invention can accept many times their own weight of pH 7 buffer solution and can be measured to be preferably below 5, even after the addition of 24 times its weight of pH 7 buffer. As set forth in Figure 7, the pH of RepHresh^{®} Vaginal Gel after addition of the corresponding quantity of pH 7 buffer is 6.32, a difference of 1.70 pH units. (Fig.1, Buffer Solution C).

The compositions of this invention may also contain other ingredients for use in emulsified, gel or two-phase systems. For example, emulsions may contain surfactants, oils, humectants, pH adjustors, waxes, polymer carriers, bioadhesives and water known to those of ordinary skill in the art. Gel formulations may contain oils, humectants, carbomers, cellulose, polyalkylene glycols and water, in addition to the active ingredients and buffer systems. The compositions may be in the form of creams, suppositories, gels or dual-phase combinations.

The two-phase dosage form of this invention is not limited to the nature or physical state of the material as pharmaceutically acceptable carrier. For example, the phase containing the antifungal azole may be solid (e.g., suppositories composed of wax-base, fat-base, polymer-base or freeze-dried) or a semi-solid (e.g., emulsion, oil-in-water cream, water-in-oil cream, ointment, or aqueous gel). Similarly, the phase containing the buffering agent(s) may also be solid or semi-solid of various pharmaceutical dosage forms. One example of such two-phase dosage form preferably contains a buffered gel and a hydrophobic antifungal component in a delivery system. The hydrophobic phase of the combination is stable inside the delivery system and is designed to melt at body temperature. Such a dosage form may be delivered, both phases together, by an applicator which is capable of insertion into the vaginal cavity. Advantageously, a two-phase dosage form permits simultaneous delivery of antifungal medication and buffering gel to the vagina, thus providing treatment capability of both mycotic and bacterial infections. The antifungal medication fights mycotic infections while the buffering gel lowers and maintains the pH of the vagina in a healthy range.

The method of using the compositions of this invention provides treatment for mycotic vulvovaginitis and bacterial vaginosis. The compositions are administered to the vaginal cavity by insertion therein. Preferably, a bioadhesive component within the compositions of this invention provides retention of the active ingredient and the buffering system in conjunction with the mucosal membranes of the vagina. The compositions may be reapplied daily until any abnormal flora, including fungus and/or bacteria, are destroyed and the infection is cured.

The following examples are merely illustrative of several of the possible compositions of this invention. Although some of the compositions in the following examples contain both antifungal azole and buffering agent(s) for co-administration into the vagina, the antifungal azole in these composition can be replaced by purified water to form buffer compositions for sequential administration as described previously. The examples serve only to illustrate, and not to limit, the compositions and methods of this invention.

### Example# 1: Hydrophilic Creams

| Ingredient | % w/w (1A) | % w/w (1B) | % w/w (1C) | % w/w (1D) |
|---|---|---|---|---|
| Stearyl Alcohol | 8.500 | 8.500 | 8.500 | 8.500 |
| Cetyl Alcohol | 3.000 | 3.000 | 3.000 | 3.000 |
| Polysorbate 60 | 3.000 | 3.000 | 3.000 | 3.000 |
| Isopropyl Myristate | 1.000 | 1.000 | 1.000 | 1.000 |
| Propylene Glycol | 20.000 | 20.000 | 20.000 | 20.000 |
| Benzoic Acid | 0.200 | 0.200 | 0.200 | 0.200 |
| Potassium Hydroxide | 0.055 | 0.055 | 0.055 | 0.055 |
| Glucono Delta Lactone (GDL) | 1.000 | 1.000 | 1.800 | 0.900 |
| Carbomer (Carbopol 974P) | -- | 2.000 | -- | 0.900 |
| Miconazole Nitrate | 4.000 | 4.000 | 4.000 | 4.000 |
| Purified Water | 59.245 | 57.245 | 58.445 | 58.445 |

The composition of this example may be prepared using the following procedure:
1. Add water and propylene glycol to a container and heat to from about 70 to about 75°C while mixing at low speed with paddle stirrer. When the mixture reaches the desired temperature(s), add benzoic acid with continuous mixing. When the benzoic acid is dissolved add potassium hydroxide and mix until dissolved.
2. When the potassium hydroxide is dissolved, add polysorbate 60 and mix for about 1 minute while maintaining the batch temperature at 70 -75°C. Then stop the mixer and add isopropyl myristate, cetyl alcohol, and stearyl alcohol. Mix the batch again at from about 70 to about 75°C until all ingredients in the container are completely dispersed.
3. Remove the container from the heat source and continue mixing using a homogenizer for about two minutes. After homogenization, mix the batch with paddle stirrer while cooling the batch to about 40°C.
4. When the temperature reaches about 40°C, add miconazole nitrate to the container with mixing. After adding miconazole nitrate, add glucono delta lactone to the container and homogenize the mixture for about four minutes or until the miconazole nitrate is completely dispersed. After homogenization, continue mixing with a paddle stirrer for about 5 minutes.

### Example# 2: Buffered Placebo Cream

| Ingredient | % w/w (2A) | % w/w (2B) |
|---|---|---|
| Stearyl Alcohol | 8.500 | 8.500 |
| Cetyl Alcohol | 3.000 | 3.000 |
| Polysorbate 60 | 3.000 | 3.000 |
| Isopropyl Myristate | 1.000 | 1.000 |
| Propylene Glycol | 20.000 | 20.000 |
| Benzoic Acid | 0.200 | 0.200 |
| Potassium Hydroxide | 0.055 | 0.055 |
| Glucono Delta Lactone (GDL) | -- | 1.800 |
| Carbomer (974) | -- | -- |
| Miconazole Nitrate | -- | -- |
| Purified Water | 64.245 | 62.445 |

The composition of this example may be prepared using the following procedure:
1. Add water and propylene glycol to a container and heat to from about 70 to about 75°C while mixing at low speed with paddle stirrer. When the mixture reaches the desired temperature(s), add benzoic acid with continuous mixing. When the benzoic acid is dissolved add potassium hydroxide and mix until dissolved.
2. When the potassium hydroxide is dissolved, add polysorbate 60 and mix for about 1 minute while maintaining the batch temperature at 70 -75°C. Then stop the mixer and add isopropyl myristate, cetyl alcohol, and stearyl alcohol. Mix the batch again at from about 70 to about 75°C until all ingredients in the container are completely dispersed.
3. Remove the container from the heat source and continue mixing using a homogenizer for about two minutes. After homogenization, mix the batch with paddle stirrer while cooling the batch to about 40°C.
4. When the temperature reaches about 40°C add glucono delta lactone if needed to the container and homogenize the mixture for about four minutes or until the miconazole nitrate is completely dispersed. After homogenization, continue mixing with a paddle stirrer for about 5 minutes.

### Example# 3: Single-Carbomer gels

| Ingredient | %w/w, placebo (3A) | % w/w, with Azole compound (3B) |
|---|---|---|
| Potassium Chloride | 0.16 | 0.16 |
| EDTA | 0.02 | 0.02 |
| Carbomer 974P (Carbopol 974P, B.F. Goodrich) | 2.08 | 2.08 |
| Sodium Hydroxide | 0.17 | 0.17 |
| Miconazole Nitrate | -- | 4.00 |
| Purified Water | 97.57 | 93.57 |

The composition of this example may be prepared using the following procedure:
1. Add Carbomer 974P into water and mix using a high speed mixer at room temperature, such as homogenizer
2. Then add potassium chloride, EDTA, and sodium hydroxide and mix using a low speed mixer, such as paddle mixer
3. For the formulation containing azole compound, add the miconazole nitrate into the mixture and mix using both homogenizer and paddle to have a uniform dispersion of miconazole nitrate in the formulation.

### Example# 4: Multi-Carbomer Gels

| Ingredient | % w/w (4A) | % w/w placebo, (4B) | % w/w (4C) | % w/w placebo, (4D) |
|---|---|---|---|---|
| Carbomer 971 | 2.00 | 2.00 | 2.00 | 2.00 |
| Mineral Oil | 4.20 | 4.20 | 4.20 | 4.20 |
| Glycerin | 12.90 | 12.90 | -- | -- |
| Carbomer 974 | 1.00 | 1.00 | 1.00 | 1.00 |
| Distilled monoglycerides | 1.00 | 1.00 | 1.00 | 1.00 |
| Sorbic Acid | 0.08 | 0.08 | 0.08 | 0.08 |
| Polyethylene Glycol 400 | -- | -- | 12.90 | 12.90 |
| Miconazole Nitrate | 4.00 | -- | 4.00 | -- |
| Purified Water | 74.82 | 78.82 | 74.82 | 78.82 |

The composition of this example may be prepared using the following procedure:
1. Add glycerin, mineral oil (or polyethylene glycol 400), distilled monoglycerides (such as Myverol), and sorbic acid into a suitable container and heat to 65-70°C. Then add Carbomer 971 and 974 into the container and mix.
2. Heat the water separately to 55-60°C and then add to the mixture from (1). Mix for about 3 minutes before adding miconazole nitrate into the container.
3. Mix the batch with a paddle stirrer while cooling down to about 45°C. When the temperature about 45°C, mix the batch using a homogenizer for about 2 minutes.
4. Switch the mixing method back to the paddle stirrer while cooling the batch to room temperature.

### Example# 5: Carboxymethylcellulose gels

| Ingredient | %w/w (5A) | %w/w (placebo) (5B) |
|---|---|---|
| Glucono Delta Lactone (GDL) | 2.50 | 2.50 |
| Sodium Hydroxide | 0.25 | 0.25 |
| Methylparaben | 0.20 | 0.20 |
| Glycerin | 17.00 | 17.00 |
| Hydroxyethylcellulose | 3.00 | 3.00 |
| Miconazole Nitrate | 4.00 | -- |
| Purified Water | 73.05 | 77.05 |

The composition of this example may be prepared using the following procedure:
1. Add Hydroxyethylcellulose into water and mix using a high speed mixer at room temperature, such as homogenizer
2. Then add glycerin, methylparaben, sodium hydroxide, and glucono delta lactone and mix using a low speed mixer, such as paddle mixer
3. Add the miconazole nitrate into the mixture and mix using both homogenizer and paddle to have a uniform dispersion of miconazole nitrate in the formulation.

### Example# 6: Adhesive/Hydrophobic Suppository

| Ingredient | % w/w (6A) | % w/w (6B) |
|---|---|---|
| Xanthan Gum | 1.00 | 1.00 |
| Sodium Carboxymethylcellulose 7HF | 8.00 | 8.00 |
| Colloidal Silicon Dioxide | 1.00 | 1.00 |
| Wecobee M | 12.00 | 15.00 |
| Wecobee FS | 54.00 | 67.00 |
| Miconazole Nitrate | 24.00 | 8.00 |

The composition of this example may be prepared using the following procedure:
1. Melt the Wecobee M and FS, (which are hard fat bases consisting primarily of mixtures of the triglyceride esters of the higher saturated fatty acids along with varying proportions of mono- and diglycerides) in a suitable container at 50 to 60°C. Add xanthan gum, colloidal silicon dioxide, and sodium carboxymethylcellulose 7HF into the container with proper mixing. Continue mixing with a homogenizer for about 2 minutes or until the additives are fully dispersed.
2. Add the miconazole nitrate into the batch while mixing with a homogenizer. Cool the batch to room temperature while mixing with a low speed mixer. The batch solidifies at temperature < 35°C.

### Example #7: Buffering Capacity

In order to determine the buffering capacity of the compositions of this invention, the following procedure was used.

The amounts of 0.1N sodium hydroxide to change the pH of samples described in Examples 1-5 were determined by a titration method. The amount of sodium hydroxide solution added to the samples in molar-equivalent basis is presented in the following graphs.

The sample produced from Example 6 contained no buffer capacity between 3.0 and 5.5 and is designed to be delivered with a placebo buffering gel (Example 5) in an applicator. This is an example of the described two-phase delivery system. Data obtained for Buffered Metrogel-Vaginal® treatment (available from 3M Corporation, Minneapolis, Minnesota)for bacterial vaginosis treatment is provided for comparison as set forth as the comparator in the Figures.

### Example 8: In Vitro Evaluation of Antibacterial Vaginosis Organism Activities

The ability of selected vaginosis anaerobes to survive in a mixture of disclosed formulations and supplemented brucella broth was also studied. The brucella broth, supplemented with vitamin K and hemin, was prepared in double strength to allow for dilution with the formulations of this invention. Studied organisms were taken from a freezer and sub-cultured at least twice to ensure purity and good growth. The following procedures were used to perform the *in vitro* evaluation:

### Method: Steer's Replicator Assay (survival time in hrs)

1. Mix the test sample 1 gram plus 9 ml dimethyl sulphoxide ("DMSO"). One of the preparations should be melted at 40-46°C and mixed thoroughly prior to dissolving in DMSO. Prepare 18 ml. Remove a small quantity and measure and record the pH. Pass into chamber and allow to become anaerobic for at least 2 hrs.
2. Working in the chamber, prepare a suspension equal to the #1 McFarland equivalence turbidity standard for each anaerobic organism in double strength-supplemented brucella broth (~3X10⁸ cfu/ml). Add 0.5 ml to the steers replicator wells. Stamp one BBA (brucella blood agar) plate as a pre- growth control.
3. Add 0.5 ml of the cream solution to the broth to each of the wells, using multichannel pipettor. Mix thoroughly by pipetting up and down. When completed, record how long it took to inoculate the entire replicator head. ( first wells will have had a longer contact time than last wells). Stamp a BBA plate as "0" time. Use one steer's replicator head for each of the creams. Each day of the test set-up, prepare a control replicator with organisms' suspensions plus brucella broth and DMSO (1 + 9), but no cream.
4. Incubate with prongs in the wells.
5. Every hour, stamp another BBA and label the plate with the time in hours. Incubate at 36°C.
6. Continue to 24 hours.
7. Examine the stamped plates after 72 hours of incubation and record if there is growth or no growth or describe type of growth i.e. few colonies, hazy growth etc. that might suggest damaged cells.
8. Final report is reported in the time in hours that the organism survived in the presence of each of the samples in Table I below.
For the azole compounds studied, miconazole, terconazole, and fluconazole are approved azoles for treating vulvovaginal candidiasis. Metronidazole and tinidazole are compounds known to be useful for treating bacterial vaginosis. However, the unexpected finding from this in vitro evaluation of azole compounds is that the miconazole actually has a better activity against bacterial vaginosis organisms than terconazole and fluconazole.

### Results of In Vitro Evaluation: Activities of Azole compounds - Table I

**Present range of MICs for each organism instead of individual rows**

| | **MIC's (µg/ml) of drug needed to inhibit the growth of organism** | | | | |
|---|---|---|---|---|---|
| **Organism** | **Metronidazole** | **Miconazole** | **Tinidazole** | **Terconazole** | **Fluconazole** |
| *Gardnerella vaginalis* | 8 | 16 | >128 | 64 | >2048 |
| *Gardnerella vaginalis* | 4 | 32 | 1 | 64 | >2048 |
| *Gardnerella vaginalis* | >32 | 16 | 128 | 64 | >2048 |
| *Gardnerella vaginalis* | >32 | >128 | 16 | 256 | >2048 |
| *Peptostreptococcus magnus* | 0.5 | 64 | 0.25 | 256 | >2048 |
| *Peptostreptococcus magnus* | 1 | 32 | 0.5 | 256 | >2048 |
| *Peptostreptococcus magnus* | 0.25 | >128 | 0.125 | 256 | >2048 |
| *Peptostreptococcus tetradius* | 1 | 128 | 0.5 | 256 | >2048 |
| *Peptostreptococcus tetradius* | 1 | 128 | 0.5 | 256 | >2048 |
| *Peptostreptococcus tetradius* | 0.5 | 16 | 0.25 | No growth | 2048 |
| *Peptostreptococcus asaccharolyticus* | 2 | 64 | 1 | 256 | >2048 |
| *Peptostreptococcus asaccharolyticus* | 0.25 | 16 | 1 | 256 | >2048 |
| *Peptostreptococcus asaccharolyticus* | 0.5 | 64 | 1 | 256 | >2048 |
| *Prevotella bivia* | 1 | 128 | 1 | 256 | >2048 |
| *Prevotella bivia* | 1 | 64 | 1 | 256 | >2048 |
| *Prevotella disiens* | 0.5 | 64 | 0.125 | No growth | >2048 |
| *Prevotella disiens* | 0.5 | 64 | 1 | 128 | >2048 |
| *Prevotella disiens* | 1 | 64 | 1 | 256 | >2048 |
| *Prevotella intermedia* | 1 | 64 | 0.5 | 128 | >2048 |
| *Prevotella intermedia* | 1 | 64 | 1 | 256 | >2048 |
| *Prevotella melaninogenica* | 1 | 64 | 2 | 64 | >2048 |
| *Prevotella melaninogenica* | 0.25 | 64 | 1 | No growth | >2048 |
| *Mobiluncus mulieris* | 4 | 8 | 1 | 256 | >2048 |
| *Bacillus fragilis* | 0.5 | >128 | 0.5 | 256 | >2048 |
| *Bacillus theta* | 2 | 128 | 1 | 256 | >2048 |
| *Lactobacillus plantarum* | 1 | 32 | 0.65 | 256 | >2048 |
| *Lactobacillus species* | > 32 | >128 | >128 | 512 | >2048 |
| *Lactobacillus acidophilus* | >32 | >128 | >128 | 512 | >2048 |
| *Lactobacillus acidophilus* | >32 | >128 | <128 | 512 | >2048 |

The activity of disclosed formulations against bacterial vaginosis organisms are shown in the following Table II. Among the formulations studied, the examples 2A, 2B, 4B, and 4D are formulations without miconazole nitrate. The example 2A which has the lowest buffer capacity, shows the least effectiveness against the studied organisms. The example 2B is the buffered placebo formulation of example 1C and the example 4D is the buffered placebo formulation of example 4C. The activity is against the studied organisms is enhanced significantly by incorporating the miconazole nitrate into the example 1C. Same results are obtained by incorporating the miconazole nitrate into the example 4D.

### Results of In Vitro Evaluation: Activities of Formulations of the Invention - Table II

| | **EXAMPLE** | | | | | | | | |
|---|---|---|---|---|---|---|---|---|---|
| **Organism** | **4B** | **5B** | **4C** | **4D** | **Monistat 3 vaginal cream** | **1C** | **2A** | **2B** | **MetroGel -Vaginal** |
| | | | | | | | | | |
| *Gardnerella vaginali*s | 2 | >7<24 | 0 | 1 | 0 | 0 | 0 | 1 | 2 |
| *Gardnerella vaginali*s | 1 | >7<24 | 0 | 3 | 0 | 0 | 0 | 2 | 4 |
| *Gardnerella vaginalis* | 1 | >7<24 | 0 | 3 | 0 | 0 | 0 | 4 | >9<23 |
| *Gardnerella vaginali*s | 3 | >7<24 | 1 | 7 | 2 | 1 | 6 | >24 | >9<23 |
| *Peptostreptococcus magnus* | 4 | >7<24 | 1 | 7 | 6 | 1 | >24 | 23 | 0 |
| *Peptostreptococcus magnu*s | 4 | >7<24 | 1 | 6 | 5 | 1 | >24 | >24 | 0 |
| *Peptostreptococcus magnu*s | 2 | >7<24 | 1 | 3 | 4 | 1 | >24 | >24 | 0 |
| *Peptostreptococcus tetradiu*s | 1 | >7<24 | 0 | 1 | 1 | 1 | >24 | >9<23 | 0 |
| *Peptostreptococcus tetradius* | 0 | >7<24 | 0 | 5 | 1 | 1 | >24 | >9<23 | 0 |
| *Peptostreptococcus tetradius* | 1 | >7<24 | 1 | 5 | 2 | 0 | >24 | >9<23 | 0 |
| *Peptostreptococcus asaccharolyticus* | 2 | >7<24 | 1 | 3 | 2 | 1 | >24 | >9<23 | 0 |
| *Peptostreptococcus asaccharolyticus* | 2 | >7<24 | 1 | 3 | 2 | 1 | >24 | 23 | 0 |
| *Peptostreptococcus asaccharolyticus* | 2 | >7<24 | 1 | 5 | 1 | 1 | >24 | >9<23 | 0 |
| *Prevotella bivia* | 2 | >7<24 | 1 | 4 | 1 | 1 | >24 | >9<23 | 0 |
| *Prevotella bivia* | 2 | >7<24 | 1 | 4 | 1 | 1 | >24 | >24 | 0 |
| *Prevotella bivia* | 2 | 6 | 1 | 8 | 1 | 1 | >24 | >9<23 | 0 |
| *Prevotella disiens* | 1 | >7<24 | 0 | 2 | 1 | 1 | >24 | 7 | 0 |
| *Prevotella disiens* | 1 | >7<24 | 0 | >24 | 1 | 0 | >24 | 4 | 0 |
| *Prevotella disiens* | 1 | >7<24 | 1 | >24 | 1 | 1 | >24 | 8 | 0 |
| *Prevotella intermedia* | 0 | 4 | 0 | >8<23 | 1 | 1 | >24 | 7 | 0 |
| *Prevotella intermedia* | 0 | 3 | 0 | >8<23 | 1 | 1 | >24 | 7 | 0 |
| *Prevotella melaninogenica* | 1 | 6 | 1 | >8<23 | 1 | 1 | >24 | >9<23 | 0 |
| *Prevotella melaninogenica* | 1 | >7<24 | 0 | 1 | 1 | 1 | >24 | >9<23 | 0 |
| *Mobiluncus mulieris* | 24 | 24 | >24 | 1 | >24 | 23 | 1 | 1 | 1 |
| *Bacillus fragilis* | >7<24 | 24 | 1 | >24 | 3 | 1 | >24 | >24 | 0 |
| *Bacillus theta* | >7<24 | 24 | 1 | >8<23 | 2 | 1 | >24 | >24 | 0 |
| *Lactobacillus plantarum* | 1 | >7<24 | 1 | 5 | 1 | 1 | >24 | 2 | 0 |
| *Lactobacillus speci*es | >7<24 | 24 | 5 | >24 | 23 | 1 | >24 | >24 | >9<23 |
| *Lactobacillus acidophilus* | 24 | 24 | >24 | >24 | >24 | 5 | >24 | >24 | >24 |
| *Lactobatillus acidophilus* | 24 | 24 | >24 | >24 | >24 | 23 | >24 | >24 | >24 |

### Example #9: Results of A pilot Clinical Study of two buffered miconazole vaginal formulations

A Phase II in vivo pilot study was conducted to evaluate the therapeutic efficacy of two preferred buffered (4%) miconazole nitrate formulations (prototypes #1 and #2) compared with MetroGel-Vaginal® gel for the treatment of bacterial vaginosis (BV) when administered intravaginally. All products were administered daily for 5 days. The efficacy parameters for this pilot study were therapeutic cure rate (combined clinical and microbiological cure), clinical cure (relief of signs and symptoms) and microbiogical cure (Nugent score of 3 or less). Therapeutic, clinical and microbiological cure rates at return office visit scheduled 21-30 days after the initial dose of treatment were similar for miconazole nitrate buffered cream and Metrogel® vaginal. Therefore the buffered miconazole cream product administered for five days appears to be effective in et treatment of bacterial vaginosis. Vulvovaginal adverse events were reported by 50-60% of miconazole-treated subjects and 21 % of Metrogel-treated subjects. Most adverse events were mild or moderate in intensity.

### Prototype# 1: Buffered Miconazole Nitrate Vaginal Cream

| Ingredient | % w/w |
|---|---|
| Stearyl Alcohol | 8.5 |
| Cetyl Alcohol | 3 |
| Polysorbate 60 | 3 |
| Isopropyl Myristate | 1 |
| Propylene Glycol | 20 |
| Benzoic Acid | 0.2 |
| Potassium Hydroxide | 0.055 |
| Glucono Delta Lactone (GDL) | 1.8 |
| Miconazole Nitrate | 4 |
| Purified Water | 58.445 |

### Prototype# 2: Buffered Miconazole Nitrate Vaginal Gel

| Ingredient | % w/w |
|---|---|
| Carbomer971 | 2 |
| Mineral Oil | 4.2 |
| Carbomer 974 | 1 |
| Distilled Monoglycerides | 1 |
| Sorbic Acid | 0.08 |
| Polyethylene Glycol 400 | 12.9 |
| Miconazole Nitrate | 4 |
| Purified Water | 74.82 |

### Example 10: Acid-Acid Buffered Compositions

The following compositions are made in a manner similar to that set forth above, however, without the inclusion of an antifungal compound. Preferably, they should be made by adding an organic acid to the polyacrylic polymer and heating to about 35° to about 50°C while mixing the formulation. The other components are then added to this mixture and the entire formulation stirred until it is homogeneous. The mixture is then cooled. Preferably, they should be made by adding an organic acid to the polyacrylic polymer and heating to about 35° to about 50°C while mixing the formulation. The other components are then added to this mixture and the entire formulation stirred until it is homogeneous. The mixture is then cooled.

### Example 10A

| Ingredient | % w/w |
|---|---|
| Mineral Oil | 1.50 |
| Benzoic Acid | 0.20 |
| MVE/MA Copolymer (Stablileze 06) | 1.50 |
| Calcium Sodium PVM/MA Copolymer (Gantrez MS-955) | 0.50 |
| Hydrogenated Palm Glyceride | 1.00 |
| (Myverol 18-04 dl-alpha Tocopherol (Vitamin E) | 0.10 |
| Propylene Glycol | 20.00 |
| Carbopol 974P | 3.50 |
| Glucono Delta Lactone | 3.00 |
| Water | Q.S.100.00 |

### Example 10B

| Ingredient | % w/w |
|---|---|
| Mineral Oil | 1.50 |
| Benzoic Acid | 0.20 |
| Calcium Sodium PVM/MA Copolymer (Gantrez MS-955) | 0.50 |
| Hydrogenated Palm Glyceride | 1.00 |
| (Myverol 18-04 dl-alpha Tocopherol (Vitamin E) | 0.10 |
| Propylene Glycol | 20.00 |
| Carbopol 974P | 3.00 |
| Lactic Acid | 1.00 |
| Water | Q.S.100.00 |

### Example 10C

| Ingredient | % w/w |
|---|---|
| MVE/MA Copolymer (Stablileze 06) | 1.50 |
| Benzoic Acid | 0.20 |
| Hydrogenated Palm Glyceride | 1.00 |
| (Myverol 18-04) | |
| dl-alpha Tocopherol (Vitamin E) | 0.10 |
| Propylene Glycol | 20.00 |
| Carbopol 974P | 4.00 |
| Lactic Acid | 3.00 |
| Water | Q.S.100.00 |

### Example 10D

| Ingredient | % w/w |
|---|---|
| dl-alpha Tocopherol (Vitamin E) | 0.10 |
| Propylene Glycol | 20.00 |
| Carbopol 974P | 4.00 |
| Lactic Acid | 3.00 |
| Hydroxyethylcellulose | 2.50 |
| Water | Q.S.100.00 |

### Example 10E

| Ingredient | % w/w |
|---|---|
| Carboxymethylcellulose | 2.00 |
| Benzoic Acid | 0.20 |
| dl-alpha Tocopherol (Vitamin E) | 0.10 |
| Propylene Glycol | 20.00 |
| Carbopol 974P | 4.00 |
| Lactic Acid | 3.00 |
| Water | Q.S.100.00 |

### Example 10F

| Ingredient | % w/w |
|---|---|
| Hydroxypropylmethylcellulose | 2.00 |
| Benzoic Acid | 0.20 |
| dl-alpha Tocopherol (Vitamin E) | 0.10 |
| Propylene Glycol | 20.00 |
| Carbopol 974P | 4.00 |
| Lactic Acid | 3.00 |
| Water | Q.S.100.00 |

### Example 10G

| Ingredient | % w/w |
|---|---|
| Propylene Glycol | 50.00 |
| Carbopol 974P | 4.00 |
| Lactic Acid | 3.00 |
| Water | Q.S.100.00 |

### Example 10H

| Ingredient | % w/w |
|---|---|
| Mineral Oil | 1.50 |
| Benzoic Acid | 0.20 |
| MVE/MA Copolymer (Stablileze 06) | 1.50 |
| Calcium Sodium PVM/MA Copolymer | 0.50 |
| (Gantrez MS-955) | |
| Hydrogenated Palm Glyceride | 1.00 |
| (Myverol 18-04 dl-alpha Tocopherol (Vitamin E) | 0.10 |
| Propylene Glycol | 20.00 |
| Carbopol 974P | 4.00 |
| Lactic Acid | 3.00 |
| Water | Q.S.100.00 |

### Example 11: pH and Buffering Capacity of Compositions

The pH and buffering capacity of the Buffer 10564-83C of this invention and that of RepHresh^{®} Vaginal Gel are summarized in Table 11A below and graphically illustrated in Figure 1. It is very clear form this data that after the addition of 130 ml of pH 7 buffer, the pH of the buffer of this invention is around pH 5, 5.02 to be exact while that of the RepHresh^{®} Vaginal Gel is higher than pH 6, 6.32 to be exact.

The pH and the buffering capacity of the compositions of this invention are illustrated in Figure 11B. These are typical Buffering Capacity plots for the two gels, Example 10A (aqueous gel) and Example 10B (aqueous gel) of the current invention. The data is also summarized in Table 11B. pH 7 buffer was added in increments of 5 ml to a 10 g sample of the product and pH was determined. The pH data clearly shows the a much lower pH was consistently maintained by the compositions of this invention throughout the successive additions of pH 7 buffer as compared with RepHresh^{®} Vaginal Gel. This means that the compositions of this invention have much lower pH profile and a much higher buffering capacity. In actual human use, the compositions of the invention are expected to be able to maintain a much more lower healthy vaginal pH as compared with RepHresh^{®} Vaginal Gel.

**Table 11A**

| Buffering Capacity of the Buffer Composition of the Invention | | |
|---|---|---|
| ml of pH 7 Buffer Used | RepHresh® Vaginal Gel | Buffer C of the Invention |
| 0 | 3.41 | 2.19 |
| 10 | 3.99 | 279 |
| 20 | 4.44 | 3.08 |
| 30 | 4.82 | 3.30 |
| 40 | 5.09 | 3.48 |
| 50 | 5.38 | 3.66 |
| 60 | 5.62 | 3.82 |
| 70 | 5.83 | 3.98 |
| 80 | 5.96 | 4.15 |
| 90 | 6.08 | 4.30 |
| 100 | 6.19 | 4.49 |
| 110 | 6.24 | 4.66 |
| 120 | 6.32 | 4.83 |
| 130 | | 5.02 |

**Table 11B**

| Buffering Capacity of The Compositions of The Invention | | | |
|---|---|---|---|
| ml of pH 7 Buffer Used | RepHresh Vaginal Gel | Example 10B of the Invention | Example 10A of the Invention |
| 5 | 3.71 | 3.11 | 3.01 |
| 10 | 3.99 | 3.24 | 3.16 |
| 20 | 4.44 | 3.43 | 3.29 |
| 30 | 4.82 | 3.62 | 3.49 |
| 40 | 5.09 | 3.84 | 3.74 |
| 50 | 5.38 | 4.06 | 3.92 |
| 60 | 5.62 | 4.26 | 4.09 |
| 70 | 5.83 | 4.48 | 4.28 |
| 80 | 5.96 | 4.65 | 4.42 |
| 90 | 6.08 | 4.83 | 4.58 |
| 100 | 6.32 | 5.19 | 4.74 |
| 110 | 6.24 | 5.19 | 4.88 |
| 120 | 6.32 | 5.36 | 5.05 |

### Example 12: Bacterial Vaginosis and Time Kill Test

### In Vitro Testing for Antibacterial Activity

*In Vitro* Time-Kill Studies were used to test the antibacterial activity of the compositions of this invention against BV causing organisms, namely, *Gardnerella, Mobilincus* and *Peptostrep.* A battery of vaginal anaerobes known to cause bacterial vaginal infections (BV), and strains of *lactobacilli* were used to determine the length of contact time required to inhibit and kill these test organisms. The results of this test are summarized in Table 12A. The results show that Example 10A of the composition of the invention kill the bacterial vaginosis-causing bacteria almost instantaneously.

These results show that the gels and suppositories of this invention are predicted to be effective to treat bacterial vaginal infections or bacterial vaginosis. They are expected to be able to minimize or eliminate vaginal odor by treating vaginosis

**Table 12A**

| **Results of *In Vitro* Evaluation: Activities of Composition of the Invention And That of RepHresh^{®} Vaginal Gel Against BV Organism** | | |
|---|---|---|
| Compounds | Composition Example 1 | *Rephresh* |
| **Organism:** | | |
| Gardnerella vaginalis | <1 | <1 |
| G. vaginalis | <1 | <1 |
| G. vaginalis | <1 | <1 |
| G. vaginalis | <1 | 1 |
| Peptostrep. magnus | <1 | 2 |
| Ps. magnus | <1 | 3 |
| Ps. anaerobius | <1 | <1 |
| Ps. anaerobius | <1 | 1 |
| Ps. tetradius | <1 | <1 |
| Ps. tetradius | <1 | 1 |
| Ps. lactolyticus | <1 | 1 |
| Ps. lactolyticus | <1 | 1 |
| Ps. asaccharolyticus | <1 | <1 |
| Ps. asaccharolyticus | <1 | 1 |
| Prevotella bivia | <1 | <1 |
| Prev. bivia | <1 | <1 |
| Prev. disiens | <1 | <1 |
| Prev. disiens | <1 | 0 |
| Prev. intermedia | <1 | 0 |
| Prev. intermedia | <1 | 0 |
| Prev. melaninogenica | <1 | 1 |
| Prev. melaninogenica | <1 | 0 |
| Lacto. jensenii | >8<24 | >8<24 |
| Lacto. jensenii | 3 | >8<24 |
| Lacto. gasseri | >24 | >24 |
| Lacto. iners | <1 | <1 |
| Mobil. mulieris | <1 | 1 |
| Mobil. mulieris | <1 | 1 |
| Mobil. curtisii | <1 | 2 |
| Mobil. curtisii | <1 | 2 |
| B. fragilis | >8<24 | >8<24 |
| B. thetaiotaomicron | 7 | >8<24 |

### Example 13: Clinical Study to Determine Vaginal pH and Perceived Vaginal Odor Control

A clinical study was performed to determine whether the compositions of this invention were capable of controlling or affecting vaginal pH and perceived vaginal odor in human subjects. One hundred and twenty-five women who completed the study were provided with one of the following products to apply vaginally:
Composition A is an aqueous gel as set forth in Example 10A; Composition B is an anhydrous gel as set forth in co-pending patent application Serial No. (Attorney Docket No. PPC 5209 filed concurrently herewith); Composition C is an anhydrous suppository as set forth in co-pending patent application Serial No. (Attorney Docket No. PPC 5209 filed concurrently herewith); and Composition D is a sample of RepHresh Vaginal Gel as described in U.S. Patent No. 6,017,521 and commercially available from Columbia Laboratories, Inc. of Livingston, NJ.

The subjects' vaginal pH was tested by vaginal examination with a pH indicator stick and pH electrode probe at baseline and at approximately 1, 6, 24,48, 72 and 96 hours following the test article application. The women were also asked to assess their self-perceived level of vaginal odor. The results of this test are set forth in Tables 13A and 13B below. Table 13A shows that Composition 10A of this invention effected a significant decrease in vaginal pH score compared with baseline. Table 13B shows that the use of Composition 10A of this invention resulted in significant decrease of self-perceived odor compared with baseline.

Another, preliminary clinical study was performed earlier than that set forth above in a significantly smaller group with the same formulations. Their use resulted in an immediately lower vaginal pH and lowered the self-perceived vaginal odor of the subjects. The plot of measured pH over time is set forth in Figure 8.

**Table 13A:**

| **Vaginal pH Levels using the pH Electrode Probe *Within-Treatment Analysis*** | | | | | | | |
|---|---|---|---|---|---|---|---|
| **HTR Code** | **Visit** | **Mean Score (std. dev.)** | | **Mean Change from Baseline (std. dev.)** | | **n** | **t-test p-value** |
| **A** | Baseline | 5.75 | (0.83) | --- | | 30 | --- |
| | 1 Hour | 3.58 | (0.72) | -2.17 | (0.92) | 30 | <0.0001¹ |
| | 6 Hours | 5.18 | (1.12) | -0.57 | (0.93) | 30 | 0.0022¹ |
| | Day 2 | 5.46 | (0.96) | -0.29 | (0.75) | 30 | 0.0435¹ |
| | Day 3 | 5.67 | (0.94) | -0.08 | (0.78) | 30 | >0.5000 |
| | Day 4 | 5.70 | (1.19) | -0.05 | (0.92) | 30 | >0.5000 |
| | Day 5 | 5.64 | (1.00) | -0.11 | (0.66) | 30 | 0.3741 |
| **B** | Baseline | 6.02 | (1.01) | --- | | 23 | --- |
| | 1 Hour | 4.94 | (1.45) | -1.08 | (0.66) | 23 | <0.0001¹ |
| | 6 Hours | 5.56 | (1.10) | -0.48 | (0.65) | 21 | 0.0030¹ |
| | Day 2 | 5.41 | (1.10) | -0.60 | (0.56) | 22 | 0.0001¹ |
| | Day 3 | 5.76 | (1.31) | -0.34 | (0.54) | 20 | 0.0100¹ |
| | Day 4 | 5.83 | (1.15) | -0.28 | (0.73) | 20 | 0.1078 |
| | Day 5 | 5.84 | (1.49) | -0.26 | (0.75) | 20 | 0.1378 |
| **C** | Baseline | 5.80 | (0.83) | --- | | 28 | --- |
| | 1 Hour | 4.48 | (0.77) | -1.32 | (0.93) | 28 | <0.0001¹ |
| | 6 Hours | 4.65 | (0.85) | -1.17 | (0.91) | 27 | <0.0001¹ |
| | Day 2 | 5.15 | (0.83) | -0.66 | (0.88) | 28 | 0.0005¹ |
| | Day 3 | 5.51 | (1.05) | -0.29 | (0.82) | 27 | 0.0772 |
| | Day 4 | 5.37 | (1.08) | -0.44 | (0.84) | 28 | 0.0108¹ |
| | Day 5 | 5.28 | (1.01) | -0.50 | (0.80) | 26 | 0.0038¹ |
| **D** | Baseline | 5.84 | (0.69) | --- | | 25 | --- |
| | 1 Hour | 4.76 | (0.69) | -1.08 | (0.67) | 25 | <0.0001¹ |
| | 6 Hours | 5.06 | (0.85) | -0.78 | (0.94) | 25 | 0.0003¹ |
| | Day 2 | 5.49 | (1.07) | -0.35 | (0.93) | 25 | 0.0760 |
| | Day 3 | 5.73 | (1.00) | -0.11 | (0.71) | 24 | 0.4581 |
| | Day 4 | 5.71 | (1.00) | -0.12 | (0.72) | 25 | 0.4003 |
| | Day 5 | 5.52 | (1.02) | -0.31 | (0.91) | 25 | 0.0979 |

| | | | | | | | |
|---|---|---|---|---|---|---|---|
| ¹ Significant decrease in score compared to baseline. | | | | | | | |

**Table 7**

| **Self-Perceived Vaginal Odor Within-Treatment Analysis** | | | | | | | |
|---|---|---|---|---|---|---|---|
| **HTR Code** | **Visit** | **Mean Score (std. Dev.)** | | **Mean Change from Baseline (std. dev.)** | | **n** | **t-test p-value** |
| **A** | Baseline | 2.77 | (0.82) | --- | | 30 | **---** |
| | 1 Hour | 2.10 | (1.01) | -0.62 | (0.86) | 29 | 0.0006¹ |
| | 6 Hours | 1.96 | (0.84) | -0.79 | (0.96) | 28 | 0.0002¹ |
| | Day 2 | 1.73 | (0.78) | -1.03 | (0.67) | 30 | <0.0001¹ |
| | Day 3 | 1.93 | (0.87) | -0.83 | (0.87) | 30 | <0.0001¹ |
| | Day 4 | 1.93 | (0.83) | -0.83 | (0.83) | 30 | <0.0001¹ |
| | Day 5 | 1.93 | (0.94) | -0.83 | (0.83) | 30 | <0.0001¹ |
| **B** | Baseline | 2.83 | (1.07) | --- | | 23 | --- |
| | 1 Hour | 2.09 | (0.92) | -0.73 | (0.94) | 22 | 0.0015¹ |
| | 6 Hours | 2.20 | (0.95) | -0.65 | (0.81) | 20 | 0.0020¹ |
| | Day 2 | 2.14 | (1.06) | -0.71 | (0.78) | 21 | 0.0005¹ |
| | Day 3 | 2.24 | (0.77) | -0.62 | (0.92) | 21 | 0.0059¹ |
| | Day 4 | 2.19 | (0.87) | -0.67 | (0.97) | 21 | 0.0049¹ |
| | Day 5 | 2.14 | (0.79) | -0.71 | (0.90) | 21 | 0.0017¹ |
| **C** | Baseline | 2.96 | (0.81) | --- | | 27 | --- |
| | 1 Hour | 2.29 | (1.01) | -0.63 | (0.97) | 27 | 0.0023¹ |
| | 6 Hours | 2.08 | (1.00) | -0.84 | (0.94) | 25 | 0.0002¹ |
| | Day 2 | 2.00 | (0.82) | -0.96 | (1.02) | 27 | <0.0001¹ |
| | Day 3 | 1.82 | (0.86) | -1.15 | (1.03) | 27 | <0.0001¹ |
| | Day 4 | 1.68 | (0.94) | -1.30 | (1.07) | 27 | <0.0001¹ |
| | Day 5 | 1.75 | (0.97) | -1.22 | (1.01) | 27 | <0.0001¹ |
| **D** | Baseline | 2.72 | (0.89) | --- | | 25 | --- |
| | 1 Hour | 2.28 | (0.94) | -0.44 | (1.00) | 25 | 0.0383¹ |
| | 6 Hours | 2.21 | (0.88) | -0.54 | (0.93) | 24 | 0.0091¹ |
| | Day 2 | 2.12 | (0.97) | -0.60 | (1.29) | 25 | 0.0289¹ |
| | Day 3 | 1.88 | (0.78) | -0.84 | (1.03) | 25 | 0.0004¹ |
| | Day 4 | 1.88 | (1.01) | -0.84 | (1.28) | 25 | 0.0032¹ |
| | Day 5 | 1.80 | (0.96) | -0.92 | (1.32) | 25 | 0.0019¹ |

| | | | | | | | |
|---|---|---|---|---|---|---|---|
| Scale: 1=None, 2=slight, 3=moderate, 4=strong, 5=very strong ¹ Significant decrease in score compared to baseline. | | | | | | | |

## Claims

1. An aqueous composition comprising an acid-acid buffer system, wherein the composition is anhydrous.

2. A composition according to claim 1, wherein said acid-acid buffer system comprises a water-soluble acidic polymer and an organic acid.

3. A composition according to claim 2, wherein the acidic polymer is a polyacrylic polymer.

4. A composition according to claim 3, wherein the polyacrylic polymer is crosslinked with allyl sucrose or allylpentaerythritol.

5. A composition according to claim 4 wherein said polyacrylic polymer is a carbomer.

6. A composition according to claim 2, wherein the organic acid is an alphahydroxy acid.

7. A composition according to claim 2 wherein said organic acid is selected from the group consisting of benzoic acid, alginic acid, sorbic acid, stearic acid, oleic acid, edetic acid, gluconodeltalactone, acetic acid, fumaric acid, lactic acid, citric acid, propionic acid, malic acid, succinic acid, gluconic acid, ascorbic acid and tartaric acid.

8. A composition according to any preceding claim, further comprising propylene glycol.

9. A composition according to any of claims 1 to 7, further comprising polyethylene glycol.

10. A composition according to any preceding claim, wherein said composition is in the form of a vaginal suppository.

11. A composition according to claim 10 wherein said composition further comprises hydrogenated vegetable oil.

12. A method of lowering vaginal pH comprising administering to an individual's vagina the composition according to any preceding claim.

13. A method of reducing self-perceived vaginal odor comprising administering to an individual's vagina the composition according to any preceding claim.
